# EUROPÄISCHE PATENTSCHRIFT

(11) **EP 2 607 475 B1**
(45) Veröffentlichungstag und Bekanntmachung des Hinweises auf die Patenterteilung: **06.11.2019**
(21) Anmeldenummer: 12198248.2
(22) Anmeldetag: 19.12.2012
(51) Int. Cl.: C12M 3/06, B01L 3/00, G01N 33/50

(54) **Monitoringzelle und Verfahren zur Analyse eines Zell- und Gewebewachstums**
Monitoring cell and method for analyzing a cell and tissue growth
Cellule de monitoring et procédé d'analyse de la croissance cellulaire et tissulaire

(30) Priorität: 23.12.2011 DE 102011057045
(43) Veröffentlichungstag der Anmeldung: 26.06.2013
(73) Patentinhaber: Fraunhofer-Gesellschaft zur Förderung der angewandten Forschung e.V., 80686 München (DE)
(72) Erfinder: Dr. Lausch, Holger, 04299 Leipzig (DE); Brand, Michael, 07743 Jena (DE); Dr. Arnold, Michael, 07743 Jena (DE)
(74) Vertreter: Oehmke, Volker

(56) Entgegenhaltungen:
- WO-A1-2007/044699
- WO-A1-2011/116921
- DE-A1-102009 039 956
- US-A- 5 190 878
- US-A1- 2007 161 106
- US-A1- 2010 015 656

## Beschreibung

Die Erfindung betrifft eine Monitoringzelle zur Analyse eines Zell- und Gewebewachstums, wie diese gattungsgemäß aus der DE 10 2009 039 956 A1 bekannt ist. Die Erfindung betrifft ferner ein Verfahren zur Analyse eines Zell- und Gewebewachstums.

Für eine Vielzahl medizinischer und werkstofftechnischer Entwicklungen und Anwendungen sind Kenntnisse über die Wechselwirkungen lebender Zellen und Gewebe mit Oberflächen und Materialien, z. B. bei der Entwicklung und Erprobung von Implantaten, von großem Interesse. Um solche Wechselwirkungen untersuchen zu können, werden eine Reihe von Untersuchungsverfahren verwendet, die aus dem Gebiet der Zellbiologie bekannt sind. In diesen Untersuchungsverfahren werden Parameter wie die Adhäsion, die Proliferation, die Migration oder die Differenzierung von Zellen, Zellverbänden, Zellschichten sowie die Entwicklung der extrazellulären Matrix (EZM) in der Regel zweidimensional und testabrechend untersucht. Testabbrechende Untersuchungen sind solche, bei denen ein Wachstum oder eine Entwicklung von Zellen oder Geweben über eine bestimmte Zeitdauer oder bis zu einem bestimmten Stadium ermöglicht wird, die Zellen oder Gewebe aber für eine Untersuchung an einem weiteren Wachstum bzw. einer weiteren Entwicklung gehindert werden. Übliche Zeitdauern sind beispielsweise 3, 6, 24 und 48 Stunden sowie 2, 5, 7, 10, 20 Tage. Nachteilig bei der Anwendung testabbrechender Verfahren ist der Verlust der Information darüber, wie die konkret untersuchten Zellen oder Gewebe sich weiter entwickelt hätten. Aus der Sicht der statistischen Auswertung solcherart Untersuchungen sind eine entsprechend große Anzahl von Versuchswiederholungen erforderlich, um hinreichend verlässliche Aussagen zu erhalten. Außerdem ist es möglich, dass sich der vorbestimmte Zeitpunkt des Abbruchs eines konkreten Tests für eine Aussage als ungünstig herausstellt, was selbst durch eine größere Anzahl von Replikaten des Tests nicht behoben werden kann.

Bei den bekannten Untersuchungsverfahren werden üblicherweise die oben beispielhaft genannten Parameter entweder isoliert voneinander oder nacheinander, jedoch nicht parallel untersucht. Ein solches Vorgehen hat dann Vorteile, wenn spezifische Aussagen zu einzelnen Parametern getroffen und Wechselwirkungen vermieden werden sollen.

Für die Entwicklung z. B. von Implantaten oder Gewebsersatzstoffen und deren Verwendungseignung im menschlichen oder tierischen Körper, ist jedoch eine komplexe Situation mit einer Vielzahl von Wechselwirkungen zu untersuchen, wie diese tatsächlich dann bei einer Verwendung des Implantats oder des Gewebsersatzstoffes in vivo vorliegt.

Ein wichtiger Umstand bei der Verwendung von Implantaten oder Gewebsersatzstoffen ist, dass bei deren Einbringung in den Körper, in ein Organ oder in ein Gewebe (fortan zusammenfassend: Gewebe) eine Spaltsituation zwischen Implantat und vorhandenem Gewebe vorliegt.

Aus dem Stand der Technik sind derzeit keine Lösungen bekannt, durch die eine in vivo auftretende Spaltsituation in vitro hinreichend realitätsnah abgebildet wird und untersuchbar ist.

Zwar werden beispielsweise in der DE 10 2009 039 956 A1 Vorrichtungen offenbart, bei denen in einem Innenraum einer Vorrichtung zur zeitlichen Zell- und Gewebeanalyse (Monitoringzelle) in den Innenraum vorspringende Wände vorhanden sind, zwischen deren Stirnflächen ein Spalt gebildet ist, durch den ein Medium, z. B. eine Nährlösung, fließt, jedoch dient diese konkrete Spaltsituation einer Lenkung von Zellen in Richtung auf eine Reaktionsfläche. In dem Innenraum angeordnete Elektroden zur Erzeugung elektrischer Felder dienen zur Ausrichtung und Lenkung der Zellen im Medium in Richtung einer definierten Analyseoberfläche.

Es ist weiterhin bekannt, dass elektrische Felder zur Untersuchung von Zellen und Geweben eingesetzt werden können. Dabei werden aus elektrischen Feldern und deren Veränderungen Informationen über sich verändernde Eigenschaften der Zellen oder Gewebe abgeleitet.

So ist aus der DE 103 45 573 A1 ein Verfahren bekannt, bei dem die Barrierefunktion einer Zellschicht durch die Bestimmung des transepithelialen oder transendothelialen elektrischen Widerstandes als Maß für die Dichtigkeit der Zell-Zell-Kontakte einer Zellschicht für den Durchtritt anorganischer Ionen ermittelt wird. Dabei werden die Elektroden ober- und unterhalb einer zu untersuchenden Zellschicht positioniert. Ist das Substrat, auf dem die Zellen adhärieren sollen, impermeabel, so werden die elektrischen Eigenschaften der Zellen impedanzspektroskopisch analysiert.

Aus der WO 2011/116921 A1 ist eine Kultivierungseinrichtung bekannt, die einen Innenraum aufweist, der von Wänden eines Gehäuses umschlossen ist. Das Gehäuse weist mindestens eine Medienzuführung an mindestens einer Seite des Innenraums und mindestens eine Medienabführung an mindestens einer anderen Seite des Innenraums sowie eine Anzahl von in dem Innenraum angeordneten Elektroden auf. In bestimmten Ausgestaltungen der Kultivierungseinrichtung können Substrate, die zur Aufnahme biologischer Zellen im adhärenten Zustand eingerichtet sind, einander gegenüber und sich gegenseitig zugewandt angeordnet sein, so dass zwischen den Substraten ein Spalt gebildet ist. Das Substrat umfasst einen Substratkörper mit einer Trägerfläche, auf der ein thermoresponsives Polymermaterial angeordnet ist. Mittels mindestens eines Temperierungselements ist mindestens auf einem Segment der Trägerfläche eine Temperatur lokal einstellbar.

Gemäß der Veröffentlichungen von Wegener et al. (2000) Brain Research 853: 115-124; Lohmann et al. (2004) Brain Research 995: 184-196; Arndt et al. (2004) Biosensors and Bioelectronics 19: 583-594, werden dabei aus den Impedanzspektren der elektrische Widerstand der barrierebildenden Zell-Zell-Kontakte, die Impedanzbeiträge des Zell-Substrat-Kontaktes und die Kapazität der Plasmamembran extrahiert. Über globale Elektroden, die ober- und unterhalb des Zellsubstrats angeordnet sind, können jedoch nur vertikale und keine horizontalen Wachstumsprozesse verfolgt werden.

Weitere Möglichkeiten zur Untersuchung des Wachstums von Zellen oder Geweben bestehen in einer Beobachtung durch geeignete Fenster mittels optischer Sensoren, wobei nur die Oberflächendynamik beobachtet werden kann. Mit der Anordnung der Zellmembranen/-träger auf einer oder zwischen zwei horizontalen Elektroden (Ring-Punkt, Platte-Platte etc.) sowie zwischen radialen Elektroden, können immer nur integrale oder eindimensionale Auflösungen von zweidimensionalen, planaren Flächen gemessen werden. Der so erzielte integrale Ausdruck löst die planare Fläche aber nicht in sich auf, was in der Regel auf die Detektion von Monolayern schließen lässt. Die Komplexität und Multilayereigenschaften beispielsweise der extrazellulären Matrix (EZM) oder von Biofilmen lässt sich so nicht räumlich und zeitlich differenziert verfolgen.

Allen vorstehend genannten Lösungen ist gemein, dass durch diese die in situ Situation zweier sich gegenüber liegender Grenzflächen mit einem dazwischen liegenden Spalt nicht abgebildet werden. Außerdem wird bei all diesen Anordnungen der gravitative Aspekt bei der Zell-/Gewebeentwicklung vernachlässigt oder durch Fluidik-Anordnungen aufgehoben, obwohl das atypische "Abflachen" von kultivierten Zellen auf dem Boden von Kulturschalen beziehungsweise auf nicht-organischen Membranen hinreichend bekannt ist (vergleiche z. B. Minuth et al. "3-D-Kulturen", Seiten 395 und 396).

Der Erfindung liegt die Aufgabe zugrunde eine Möglichkeit zur Analyse eines Zell- und Gewebewachstums vorzuschlagen, bei der eine in-vivo Spaltsituation zwischen den Oberflächen eines Trägerobjekts und eines Zellsubstrats nachgebildet ist.

Die Aufgabe wird durch eine Monitoringzelle zur Analyse eines Zell- und Gewebewachstums mit einem einen Innenraum umschließenden Gehäuse mit Wänden und mindestens einer Grundplatte, wobei das Gehäuse mit mindestens einer Medienzuführung an mindestens einer Seite des Innenraums und mindestens einer Medienabführung an mindestens einer anderen Seite des Innenraums und mit einer Anzahl von in dem Innenraum angeordneten Elektroden versehen ist, gelöst. Kennzeichnend für die erfindungsgemäße Monitoringzelle ist, dass mindestens eine Aufnahmevorrichtung zur Aufnahme mindestens eines Trägerobjektes und mindestens eine Aufnahmevorrichtung zur Aufnahme mindestens eines Zellsubstrats oder mindestens eine Aufnahmevorrichtung zur Aufnahme mindestens eines Trägerobjektes und mindestens eines Zellsubstrats vorhanden ist. Mindestens zum Startzeitpunkt der Analyse ist zwischen Trägerobjekt und Zellsubstrat ein Spalt vorhanden, so dass eine in vitro abzubildende in vivo Situation zwischen einem zu besiedelnden Trägerobjekt und einem Substrat nachgebildet ist. Die Elektroden sind sowohl zur Erzeugung räumlich und zeitlich definierter elektromagnetischer Felder als auch als Mittel zur Messung elektrischer Größen (fortan in der Beschreibung: Messmittel) ausgestaltet.

Nachfolgend werden unter dem Begriff des Wachstums von Zellen oder Geweben alle mit einem Wachstum verbundenen Prozesse wie z. B. Zellmigration, Differenzierung und Proliferation verstanden. Mit der erfindungsgemäßen Monitoringzelle können daneben auch zu einem Wachstum komplementäre Prozesse wie das Absterben und der Abbau von Zellen (Apoptose, Supression) sowie Veränderungen des Zellsubstrats und des Trägerobjekts analysiert werden.

Ein Spalt im Sinne dieser Beschreibung kann ein räumlicher Spalt sein. Dieser liegt vor, wenn Trägerobjekt und Zellsubstrat tatsächlich voneinander beanstandet angeordnet sind. Unter einem Spalt ist auch ein stofflicher Spalt zu verstehen. Ein stofflicher Spalt kann beispielsweise durch biologische (bioinerte, z. B. zytotoxische Bereiche), (bio-)physikalische Eigenschaften wie starke physikalische Gradienten oder materialspezifische Gradienten oder Materialeigenschaften, z. B. ein abrupter Wechsel von Oberflächenrauigkeiten, gegeben sein. Alle Grenzflächensituationen, die eine Spaltwirkung aufweisen, fallen unter den Begriff des Spalts.

Unter einem Trägerobjekt wird jeder Körper aus jedem Material verstanden, der in die Aufnahmevorrichtung eingebracht werden kann. Vorzugsweise sind Trägerobjekte aber biodegradierbare oder nicht-biodegradierbare Implantate oder Gewebeersatzstoffe. Ein Zellsubstrat kann jedes Material oder Stoffgemisch sein, durch welches Zellen bereitgestellt sind (Beispiele: besiedelte oder beimpfte Nährmedien wie Agarose, Alginat, Mono- und Kokulturen). Die erfindungsgemäße Monitoringzelle ist auch zur Analyse von Biofilmen, die beispielsweise durch Bakterien, Pilze, Algen und Protozoen gebildet sind, verwendbar. Trägerobjekte und Zellsubstrat können im Verlauf des Wachstums mit einer großen Zahl von Zellen überdeckt werden, zwischen denen sich auch EZM ausbilden kann oder sich Gewebe differenzieren. Nachfolgend ist vereinfachend von Zellen die Rede, wobei aber alle möglichen zellbiologischen Strukturen und Einheiten sowie ein- und mehrzellige Organismen umfasst sein sollen. Trägerobjekt und Zellsubstrat sind durch die jeweilige Aufnahmevorrichtung gehalten und fixiert.

Die Begriffe bio-degradierbar und degradierbar bedeuten, dass das betreffende Material durch biochemische Reaktionen des Gewebes (z. B. enzymatisch) sowie gegebenenfalls in Wechselwirkung mit biophysikalischen Prozessen abgebaut wird.

Eine bevorzugte Ausführung der erfindungsgemäßen Monitoringzelle ist so gestaltet, dass in dem Innenraum zwei Vorsprünge mit Stirnflächen vorhanden sind und die Aufnahmevorrichtungen an den Stirnflächen angeordnet sind.

Außerhalb des Spalts können, vorzugsweise in dem Innenraum, weitere Messmittel angeordnet sein. Die weiteren Messmittel können z. B. Temperatur-, Druck-, Sauerstoff- oder pH-Wert-Sensoren sein.

Es ist auch vorteilhaft, wenn eine Auswerte- und Speichereinheit vorhanden ist, die mit den Elektroden und vorhandenen weiteren Messmitteln verbunden ist. Durch eine solche Auswerte- und Speichereinheit sind die von den Messmitteln erfassten Messdaten sowie die von den als Messmittel fungierenden Elektroden, vorzugsweise zentral, auswertbar und speicherbar.

Elektroden können als elektrisch leitende, nicht-isolierte Elektroden ausgeführt sein, wodurch ein Stromfluss zwischen mindestens zwei Elektroden und durch das Medium ermöglicht ist. Mittels nicht-isolierter Elektroden sind auf Stromfluss beruhende Messungen durchführbar. Sind die Elektroden elektrisch isoliert gestaltet, können mit diesen elektrische und / oder elektromagnetische Felder in dem Innenraum erzeugt und zur Messung verwendet werden. Die Elektroden können in verschiedenartigen Formen ausgebildet, z. B. streifen-, platten- oder punktförmig oder zu sogenannten Clustern zusammengefasst, sein. Die Elektroden können fest angeordnet oder frei in dem Innenraum positionierbar sein. Dadurch ist vorteilhaft eine hohe Flexibilität bei der Gestaltung der Messanordnungen gegeben. Es können mehrere nicht-isolierte Elektroden, mehrere isolierte Elektroden und Kombinationen von nicht-isolierten und isolierten Elektroden angeordnet sein. Von Vorteil ist es, wenn die Form, Anordnung und die Art und Weise der Erzeugung der elektromagnetischen Felder eine ortsaufgelöste Erfassung der Messgrößen erlaubt. Die Elektroden können auch segmentiert werden. Dies kann beispielsweise durch eine räumlich definierte Anordnung einer Anzahl von Elektroden erreicht sein, wobei aus den Unterschieden der Messgrößen je Elektrode und der Kenntnis der Anordnung der jeweiligen Elektroden ein Ort der Erfassung der Messgrößen ableitbar ist.

Die in der Anordnung vorgesehenen Elektroden können in mehrerlei Hinsicht variiert und unterschieden werden. Zum einen hinsichtlich Ihrer Verbindung mit der leitfähigen Nährlösung/Medium in leitende (nasse) und nichtleitende (trockene) Elektroden in Gestalt elektrisch isolierter Elektroden wie auch als elektrisch leitende, galvanische Elektroden. Die Elektroden sind zum anderen hinsichtlich ihrer Geometrie, z. B. in Punkt- , Linien- und Flächenelektroden sowie ihrer Anordnung untereinander z. B. in Einzelelektrodenanordnungen, Rasteranordnungen (Clusterelektroden) und freie Anordnungen unterscheidbar. Weiterhin lassen sich theoretisch beliebige Kombinationen der vorgesehenen Elektroden zur Anregung z. B. der Zellen und/oder für Messung verwenden.

Eine bevorzugte Anordnung der Elektroden ist, wenn diese auf mindestens zwei Seiten neben den Stirnflächen der Vorsprünge angeordnet sind. Mittels einer solchen Anordnung können elektrische Felder auf beiden Seiten des Spalts, z. B. zuflussseitig und abflussseitig, erzeugt sein. Auch ist es möglich, dass mindestens eine Elektrode als zentrale Elektrode auf mindestens einer Stirnfläche angeordnet ist. Die zentrale Elektrode befindet sich dann hinter dem Trägerobjekt oder dem Zellsubstrat, so dass ein von der zentralen Elektrode ausgehendes elektrische Feld oder ein Stromfluss zwischen der zentralen Elektrode und mindestens einer weiteren Elektrode durch das Trägerobjekt beziehungsweise das Zellsubstrat hindurch erfolgt. Es können einige oder alle Elektroden verstellbar sein, so dass Elektroden dreidimensional im Innenraum angeordnet werden können. Es können auch zentrale Elektroden auch jeder der Stirnflächen vorhanden sein, so dass eine Messung über die Spaltbreite möglich ist. Die zentralen Elektroden können in Richtung auf den Spalt verstellbar sein.

Eine günstige Ausgestaltung der erfindungsgemäßen Monitoringzelle besteht darin, dass in dem Innenraum Strömungsbarrieren angeordnet sind, durch deren Anordnung das Medium durch den Spalt geleitet ist. Dadurch ist eine sichere Benetzung und Versorgung der Zellen unterstützt. Eine Strömung kann das Zellsubstrat lateral umströmen sowie in den Spalt eintreten oder durch diesen fließen. Eine Versorgung der lateralen Bereiche des Zellsubstrats mit z. B. Nährstoffen erfolgt dabei über die lateral angeströmten Bereiche, der zentrale Bereiche des Zellsubstrat ist über den Spalt versorgbar. Ebenfalls der Strömungslenkung dient es, wenn jeder Vorsprung unter einem zur Stirnfläche spitzen Winkel abfallende Flanken aufweist. Es ist auch möglich, dass das Medium mit einem bestimmten Druck in den Innenraum oder den Spalt eingeleitet ist, wodurch eine bestimmte Druckbelastung der Zellen simulierbar ist. Damit kann die Monitoringzelle an ein definiertes Pumpen- und Reservoir- sowie an ein Mikrotröpfchenentnahmesystem angeschlossen werden, um die Nährstoff-, Sauerstoff- oder Signalstoffversorgung, oder Kombinationen dieser, im Spalt zwischen Trägerobjekt und Zellsubstrat zu gewährleisten sowie andere biologische Organismen gezielt in den Spalt einbringen zu können. Das Medium kann kontinuierlich oder diskontinuierlich zugeführt sein.

Außerdem ist mit einer solchen Anordnung eine definierte Druck- und Strömungsbelastungssituation im Spaltbereich temporär, periodisch oder dauerhaft, beispielsweise für mechanotransduktive Simulationen von Belastungs- und Bewegungssituationen, erzeugbar. Es sind definierte Gradienten (z. B. Druck, Kraft, Oberflächenspannung) erzeugbar, die zu einer in situ Situation bei körperlicher Belastung vergleichbar sind.

Als vorteilhafte Ausführung der erfindungsgemäßen Monitoringzelle hat es sich erwiesen, wenn die Monitorzelle mit einem gesteuerten Antrieb verbunden ist, mittels dem die Monitorzelle gesteuert bewegbar ist. Mit einer solchen Ausführung sind zwei- oder dreidimensionale Bewegungen der Monitoringzelle realisierbar. Beispielsweise kann die Monitoringzelle definiert wipp-, kipp-, taumel-, ping-pong- und kreisschüttelbar sein, um Zell- und Gewebewachstumsprozesse sowie auch die Biofilmentwicklung unter Ruhebedingungen sowie unter definierten Bewegungssituationen simulieren zu können. Auch können Einflüsse der Gravitation auf das Wachstum oder dessen Suppression aufgehoben sein. Damit ist eine realitätsnahe Nachbildung der in vivo Situation möglich.

Um eine Spaltbreite des Spalts einstellen zu können, ist es günstig, dass mindestens eine der Aufnahmevorrichtungen in Richtung auf den Spalt verstellbar ist.

Die einzustellende Spaltbreite hängt von den jeweils zu analysierenden Situationen, Trägerobjekten und Zellsubstraten ab. So ist eine Spaltbreite zwischen 150 und 400 µm bei der Analyse des Wachstums von humanen Knochenzellen (Osteoblasten) günstig. Die gewählte Spaltbreite hängt von den zu analysierenden Kombinationen von Trägerobjekt und Zellsubstrat sowie davon ab, für welchen Organismus die Situation in vitro nachgebildet wird. So können für große Organismen, z. B. Pferde oder Kamele, andere Spaltbreiten gewählt und eingestellt sein als für kleinere Organismen wie z. B. für eine Maus. Die Spaltbreite ist vorzugsweise kleiner als 3 mm einstellbar. In einer vorteilhaften Ausgestaltung der Monitoringzelle ist die Spaltbreite auch während einer Analyse nachjustierbar, so dass beispielsweise medizinisch indizierte und definiert vorgenommene Streckungen mit einem Implantat versorgter Gewebe nachbildbar sind.

Durch eine Unterteilung des Trägerobjekts in Teilträgerobjekte und des Zellsubstrats in Teilzellsubstrate ist die Analyse mehrerer Wachstumsprozesse in einer Monitoringzelle möglich. Die Unterteilung kann auch dem Mitführen von Kontrollen sowie der Analyse von Proben im Sinne einer experimentellen Versuchsanordnung nach wissenschaftlichen und statistischen Standards (z. B. zur Analyse mittels Varianzanalyse) dienen. Die einzelnen Teilträgerobjekte oder Teilzellsubstrate können aneinander angrenzen oder durch einen Spalt oder eine Barriere, die nicht besiedelbar (bioinert, z. B. zytotoxisch) ist, voneinander getrennt sein.
Die Unterteilungen von Trägerobjekt und Zellsubstrat sind vorzugsweise zueinander in Form, Größe und Lage komplementär, so dass sich die Unterteilungen in der Monitoringzelle gegenüber stehen. Es ist auch möglich, dass mindestens eine von zwei sich gegenüber liegenden Unterteilungen neutral, d.h. nicht besiedelt oder nicht besiedelbar, ist.

Auf den Teilzellsubstraten können z. B. unterschiedliche Zellen, Co-Kulturen oder Reservoirs für Mikroben (Pro- und / oder Eukaryoten), Bakterien oder Pilze sowie Kombinationen davon vorhanden sein.

Die Trägerobjekte, das Zellsubstrat oder beide können als bereits zell- oder gewebebesiedelte Elemente ausgebildet werden, wobei diese als Biosensoren für über das Medium definiert einbringbare biologische, biochemische oder chemische Stoffe, Elemente, Isotope oder Verbindungen nutzbar sind.

Es ist im Sinne der Erfindung möglich, das Trägerobjekt und das Zellsubstrat mittels der Aufnahmevorrichtungen so zu positionieren, dass Trägerobjekt und Zellsubstrat nebeneinander liegen und der Spalt zwischen Trägerobjekt und Zellsubstrat gebildet ist.

Durch eine entsprechend gewählte Anordnung der Elektroden ist eine Analyse der so angeordneten Trägerobjekte und Zellsubstrate möglich.

Es ist beispielsweise möglich, eine von dem Zellsubstrat in einer Ebene ganz oder teilweise umgrenzte zentrale Elektrode anzuordnen. Zwischen dem Zellsubstrat und der zentralen Elektrode kann eine Barrierezone vorhanden sein. Gegenüber der zentralen Elektrode kann durch einen Spalt getrennt eine weitere zentrale Elektrode oder mehrere Elektroden angeordnet sein. Mindestens in dem Spalt ist ein Medium vorhanden.

Die Aufgabe wird ferner durch ein Verfahren zum nicht-testabbrechenden, räumlichzeitlichen in vitro - Monitoring des Wachstums von Zellen oder Geweben gelöst. Das erfindungsgemäße Verfahren umfasst die Schritte des Aufnehmens eines Trägerobjekts in einer Aufnahmevorrichtung einer erfindungsgemäßen Monitoringzelle und des Aufnehmens eines Zellsubstrats in einer weiteren Aufnahmevorrichtung der Monitoringzelle, so dass zwischen dem Trägerobjekt und dem Zellsubstrat ein Spalt mit einer Spaltbreite vorliegt.
Es wird ein Medium zugeführt, das der Benetzung und Versorgung der Zellen des Zellsubstrats und aller sich während des Wachstums entwickelnden Zellen dient. Das Medium kann den Innenraum teilweise oder ganz ausfüllen. In der erfindungsgemäßen Monitoringzelle werden räumlich und zeitlich definierte elektrische und / oder elektromagnetische Felder erzeugt und Messgrößen der elektrischen und / oder elektromagnetischen Felder erfasst und gespeichert.

Bei dem erfindungsgemäßen Verfahren werden bevorzugt Spaltbreiten kleiner als 3 mm eingestellt.

Eine bevorzugte Führung des erfindungsgemäßen Verfahrens besteht darin, dass initiale Messgrößen zu einem initialen Messzeitpunkt erfasst und gespeichert werden und die initialen Messgrößen als Referenzmessgrößen für zu späteren Messzeitpunkten erfassten Messgrößen verwendet werden. So kann eine gemessene Ist-Situation mit einer bestimmten Ausgangssituation verglichen werden und mindestens eine zeitliche Veränderung des Wachstums und der mit diesem einhergehenden Zunahme, Stagnation oder gar Abnahme der Anzahl der Zellen detektiert werden.

Aus den Veränderungen der zu verschiedenen Messzeitpunkten erfassten und gespeicherten Messgrößen können Informationen über das Wachstum der Zellen oder Gewebe abgeleitet werden. Es ist eine vorteilhafte Ausführung des erfindungsgemäßen Verfahrens, wenn die Messgrößen ortsaufgelöst erfasst werden. Die ortsaufgelöst erfassten Messdaten können graphisch dargestellt werden und die Informationen über das Wachstum der Zellen oder Gewebe aus der graphischen Darstellung abgeleitet werden. Beispielsweise kann eine ortsaufgelöste Messgröße einem oder mehreren Bildelementen (Pixel) einer Anzeige zugeordnet werden und der Wert der Messgröße kann einem Helligkeits- oder Farbwert, z. B. einem Grauwert, (Pixelwert) zugeordnet werden. Die Verteilung der Pixelwerte kann als Datensatz für eine bildgestützte Analyse dienen. Liegen Messgrößen von verschiedenen Messzeitpunkten vor, können auch die Differenzen der Pixelwerte zu den Messzeitpunkten als Datensatz verwendet werden.

Die abgeleiteten Informationen können mit Einträgen einer Datenbank abgeglichen, evaluiert und validiert werden. Die Datenbanken können bereits vorhandene Datenbanken, während der Analyse generierte Datenbanken oder Kombination von vorhandenen und generierten Datenbanken sein.

Mit der erfindungsgemäßen Monitoringzelle und dem erfindungsgemäßen Verfahren ist es besonders vorteilhaft möglich, eine Eignung von verschiedenen Materialien oder Materialverbünden für die Herstellung von Implantaten für individuelle Patienten zu untersuchen. Dazu werden Trägerobjekte aus den zu untersuchenden Materialien / Materialverbünden hergestellt und in einer erfindungsgemäßen Monitoringzelle zur Durchführung des erfindungsgemäßen Verfahrens verwendet. Dabei können neben den Materialien der Trägerobjekte auch deren Form und / oder Oberflächenstruktur (z. B. Rauhigkeit, Porosität, chemisch und / oder physikalisch auf der Oberfläche aufgebrachte Substanzen) variiert und untersucht werden. Mittels dieser Vorgehensweise kann eine möglichst optimale individuelle Kombination von z. B. durch Material und Oberflächenstruktur bestimmte Eigenschaften eines Implantats und einem Patienten gefunden werden.

In einer vorteilhaften Ausführung ist es möglich, Zellen (autologe Zellen) eines bestimmten Patienten (autologe Zellen) in dem erfindungsgemäßen Verfahren als Zellsubstrat zu verwenden. Das Trägerobjekt kann in einer ersten Ausführung aus einem ausgewählten Material mit ausgewählten Eigenschaften gebildet sein. Anhand der Veränderungen der zu verschiedenen Messzeitpunkten erfassten und gespeicherten Messgrößen werden Informationen über das Wachstum der Zellen oder Gewebe abgeleitet. Durch einen Vergleich von Informationen, die bei der Verwendung verschiedener Trägerobjekte erlangt wurden, ist eine Auswahl eines Materials bzw. eines Materialverbundes ermöglicht, welches eine Besiedlung mit den z. B. autologen Zellen in einer erwünschten Weise ermöglicht. Beispielsweise kann eine Dynamik, eine Qualität (z. B. eine Gleichmäßigkeit der Besiedlung) und / oder eine Quantität (z. B. eine Besiedlungsdichte) als Kriterium für die Auswahl herangezogen werden.

Eine weitere Ausführung der erfindungsgemäßen Monitoringzelle und des Verfahrens im Sinne dieser Erfindung erlaubt einen zeitgleichen (simultanen) Test mehrerer Trägerobjekte. Dazu können gleichzeitig mehrere Trägerobjekte in der Monitoringzelle angeordnet werden. Es ist auch möglich, das Trägerobjekt in eine Anzahl von Teilträgerobjekten zu unterteilen. Diese Teilträgerobjekte können verschiedene Eigenschaften aufweisen. So können die Teilträgerobjekte aus unterschiedlichen Materialien bestehen und / oder verschiedene Eigenschaften besitzen. Es können auch mindestens zwei Teilträgerobjekte aus gleichen Materialien bestehen und / oder gleiche Eigenschaften aufweisen. Auf diese Weise ist beispielsweise ein testinternes Replikat geschaffen.

Die Teilträgerobjekte können mit Teilzellsubstraten kombiniert werden. In einer Ausführung kann einem jeden Teilträgerobjekt ein Teilzellsubstrat zugeordnet werden. Es ist auch möglich, mindestens zwei Teilträgerobjekten ein Teilzellsubstrat zuzuordnen.

Zusätzlich zu den beispielhaft angeführten Kombinationsmöglichkeiten von (Teil-)trägerobjekte(-n) und (Teil-)zellsubstrat(-en) können auch die Eigenschaften des Mediums, z. B. dessen Zusammensetzung, pH-Wert, Sauerstoffgehalt, Temperatur etc. variiert werden.

Mittels der beschriebenen Ausgestaltungen der erfindungsgemäßen Monitoringzelle und des erfindungsgemäßen Verfahrens ist eine Verwendung der Erfindung zur Ermittlung von bestmöglichen Kombinationen von Materialien, Materialverbünden sowie deren Eigenschaften mit Eigenschaften von Zellen von individuellen Patienten in äußerst vorteilhafter Weise ermöglicht. Besonders von Vorteil ist dabei die Möglichkeit einer realitätsnahen Nachbildung einer in vivo-Situation mittels der Monitoringzelle und die dadurch ermöglichte realitätsnahe und individuelle Testmöglichkeit verschiedener Materialien von Trägerobjekten.

Es ist möglich, das Wachstum der Zellen durch eine Einbringung von Stimuli in den Innenraum zu beeinflussen. Solche Stimuli können chemische Substanzen, die eine stimulierende Wirkung auf das Wachstum haben, wie beispielsweise Wachstumsfaktoren, Nährstoffe, Hormone sowie Energieäquivalente. Günstig ist es, mit dem Medium solche chemischen Substanzen zuzuführen, wie diese auch bei dem nachgebildeten in vivo System zu einem bestimmten Zeitpunkt vorliegen würden. Dadurch ist in äußerst vorteilhafter Weise auch eine in vitro Nachbildung der sich über die Zeit verändernden biochemischen Gegebenheiten möglich.

Die chemischen Substanzen können auch andere Substanzen sein, durch die auch negative oder keine Wirkungen auf das Wachstum bewirkt sein können. Es ist möglich, das Medium zuzuführen, um das Medium regelmäßig zu erneuern, in seinen Eigenschaften definiert verändertes Medium oder verschiedene Medien zuzuführen. Eigenschaften können insbesondere die Zusammensetzung des Mediums sein. Weiterhin ist es möglich, definiert physikalische Wirkfaktoren wie mechanische, elektrische, elektromagnetische oder magnetische Felder sowie Kombinationen dieser, in den Innenraum einzukoppeln. Es können Gradienten physikalischer Wirkfaktoren in dem Innenraum erzeugt sein.

Mittels der Elektroden, welche elektrisch isoliert oder leitend zueinander variabel verschaltet und frequenzbasiert angeregt werden können, sind über Veränderungen der gemessenen elektrischen Signale (z. B. Widerstände, Leitfähigkeiten, Potentiale, Kapazitäten) Migration, Adhäsion, Proliferation, Ausdifferenzierung und Ausbildung einer extrazellulären Matrix (EZM), die Bildung von Mono- und Multilayern, Maturierung oder die Biofilmbildung, Suppression und andere zelluläre Prozesse zu monitoren und zu dokumentieren.

Die erfindungsgemäße Anordnung dient der Durchführung eines nicht-testabbrechenden, mehrdimensionalen Verfahren zur Analyse, Überwachung und Dokumentation von in vitro Zellwachstum in einer definierbaren, dreidimensionalen Grenzflächensituation zwischen einem gewebeähnlichen Bereich (Zellsubstrat) und einem Bereich in Gestalt von degradierbaren bzw. nicht degradierbaren Implantatoberflächen und oder Gewebeersatzstoffen (Trägerobjekt); mit anderen Worten für ein Monitoring von Wachstumsprozessen in einem System, durch das die in situ Situation nachgebildet ist. Es können medizinisch anzuwendende Materialien auf ihre bioaktiven sowie antiinflammatorischen Eigenschaften bezüglich humaner, tierischer (z. B. Osteoblasten, Endothelzellen, Plattenepithlien, Fibroblasten) oder sonstiger Zellen (z. B. Bakterien, Pilze, sonstige Biofilm-Bildner und sonstige Mikroorganismen) untersucht werden.

Das Monitoring von Zellwachstumsprozessen in einer in vivo- / in situ-ähnlichen Situation erfolgt über ortsbezogene Messungen der zeitlichen Veränderung elektrischer Parameter mittels impedanzspektroskopischer Messmethoden sowie kapazitiver und Potentialmessungen. Die gesamte erfindungsgemäße Anordnung ist während einer Analyse in ihrem Grundaufbau unveränderlich und mit definierten Elektrodenanordnungen versehen. In dieser initialen Bestückung, inklusive des Zellsubstrat, des Trägerobjekts sowie des Mediums, weist die Anordnung bei Messung an unterschiedlichen Elektrodenkombinationen, respektive an unterschiedlichen Orten der Elektroden, ein jeweils unterschiedliches, jedoch definiertes und reproduzierbares initiales elektrisches Verhalten auf. Dieses ist bedingt durch die in der Anordnung bzw. bei der Bestückung verwendeten Materialien, deren elektrischer Eigenschaften, deren Grenzflächen untereinander sowie deren geometrischer Anordnung zueinander. In Summe legen diese Parameter die Potentialverteilung bzw. die Signalwege bei elektrischer Anregung der Anordnung mit definierter Gleichspannung oder vorzugsweise sinusförmiger Wechselspannung fest und resultieren im Allgemeinen in einem typischen orts-, amplituden- und frequenzabhängigen Antwortverhalten.
Durch die Zellwachstumsprozesse bedingte Effekte führen im zeitlichen Verlauf des Tests zu Veränderungen der geometrischen Anordnung der Materialien (z. B. bei der Migration von Zellen oder dem Abbau des Trägermaterials) bzw. zur Veränderung der Materialien an sich (erfassbare Auswirkungen auf die Zusammensetzung des Mediums) und damit zur Veränderung der elektrischen Signalwege und/oder der elektrischen erfassbaren Messgrößen wie der Leitfähigkeit der Materialien. Die angeführten Veränderungen äußern sich in Bezug auf die genutzten Elektrodenanordnungen hinsichtlich des elektrischen Verhaltens der Anordnung in der Monitoringzelle. Aus dem zeitlichen Verlauf der Differenzen zwischen initialem elektrischen Verhalten und aktueller Messung und/oder zwischen zeitlich aufeinanderfolgenden Messungen und daraus abgeleiteten Größen wie Anstiegsverhalten oder Veränderung der Frequenz- und Amplitudenabhängigkeiten und/oder durch das Fitten (das Anpassen) der aktuellen Messung an eine elektrische Ersatzschaltung für die Anordnung, welches die Übergangswiderstände und -kapazitäten der Materialien und Medien in geeigneter Weise berücksichtigt, können Rückschlüsse auf die Zellwachstumsprozesse wie Migration- und Adhäsionsverhalten sowie Proliferation oder Suppression gezogen werden. Durch die Korrelation der elektrischen Messergebnisse mit denen etablierter zellbiologischer Untersuchungen können erstmals definiert sinnvolle Zeitpunkte festgestellt werden, zu denen die untersuchten Proben testabbrechend etablierten zellbiologischen Untersuchungen zur weitergehenden Bewertung zugeführt werden. Außerdem können auch begleitende, nicht-testabbrechende Untersuchungen (z. B. Mikrotröpfchenanalysen) sinnvoll geplant werden und/oder Modifikationen der Umgebungsbedingungen oder anderer Testparameter vorgenommen werden, beispielsweise die zielgerichtete Steuerung des Nährstoffeintrags über das Medium in den Spalt.

Zur Anregung der Anordnung können sowohl definierte Einzelfrequenzen als auch ganze Bänder von Frequenzen im Sinne eines Frequenzdurchlaufs sowie Gleichsignale verwendet werden. Die anregenden Signale können außerdem in ihren Amplituden und ihrer Signalform variiert werden.

Vorteilhafterweise werden dazu die vorhandenen Elektroden, genauer die von diesen erzeugten elektrischen und / oder elektromagnetischen Felder und die über die Elektroden erfassten Messgrößen derart kombiniert, dass beispielweise Messungen entlang einer Anordnung von Trägerobjekt und Zellsubstrat oder eine Messung durch das Trägerobjekt, durch das Zellsubstrat oder durch beide ermöglicht sind. Auch sind unterschiedliche Elektrodenkombinationen für Anregung und Messung möglich.

Als Messgrößen zur Charakterisierung beispielsweise von Zellwachstums- und Zellmigrationsprozessen können wahlweise Impedanzen in Form eines Amplitudenverhältnisses zwischen Spannung und Strom und Phasenwinkel beider Größen, komplexe Leitwerte sowie alle daraus abgeleiteten Größen, insbesondere auch elektrodenbezogene Kapazitäten, Induktivitäten, ohmsche Widerstände oder Leitfähigkeiten von Materialien und Materialkombinationen benutzt werden.

Außerdem ist es möglich, aus dem Antwortverhalten des Systems bei Messung zwischen jeweils definierten Elektroden als Bezugselektroden und variabel gewählten Elektroden unter Einbeziehung der Anordnung, der Geometrie und des Ortes von Bezugs- und Gegenelektroden mit Hilfe von Projektions- und Rekonstruktionsalgorithmen graphische 2D- bzw. 3D-Darstellungen der zellbiologisch induzierten Intensitäten bzw. Intensitätsschwankungen der elektrischen Parameter zu generieren. Diese können beispielweise in farb- oder grauwertkodierte Bilder und/oder 3D-Punktwolken überführt und mit etablierten Bildverarbeitungsalgorithmen und/oder statistischen Verfahren (z. B. Clusteranalysen) weiter untersucht werden. Auf diese Weise kann das Messverfahren als bildgebendes Verfahren für zellwachstumsbedingte Prozesse, wie beispielsweise der Lokalisierung von Besiedlungszonen auf einem Trägerobjekt oder von biochemischen Anreicherungs- und Verarmungszonen der Nährstofflösung oder des Materials des Trägerobjekts benutzt werden. Zur Generierung der ortsbezogenen Darstellungen werden vorzugsweise Elektroden mit räumlicher Affinität zum Point-Of-Interest (POI) als Bezugselektroden gewählt, beispielweise zentrale Elektroden in der Nähe des Trägerobjekts. Als variable gewählte Elektroden dienen vorzugsweise Elektroden, die einen Signalfluss in unterschiedliche Richtungen vom POI aus zulassen, beispielsweise seitlich der Stirnfläche des Vorsprungs angeordnete Elektroden, um die Anwendung verschiedener Rekonstruktions- und Projektionsalgorithmen zu gewährleisten.
Alternativ können auch über definierte, jedoch variable Elektrodenpaare definierte Ströme in die Anordnung eingeprägt und durch eine Vielzahl von Elektroden die Potentialverteilung in einer Region-Of-Interest (ROI) bestimmt werden. Auf diese Weise sind eine Rekonstruktion der örtlichen Leitfähigkeitsverteilung in der ROI sowie deren graphische Darstellung ähnlich der elektrischen Impedanz-Tomographie möglich.

Ein Schema einer möglichen messtechnischen Realisierung des Monitoring-Systems ist nachfolgend umrissen. Die einzelnen Elektroden der Monitoringzelle sind mit einem Multifunktions-Switch oder einer Schaltmatrix verbunden. Dieses Schaltmittel realisiert die weiter oben beschriebene Kombination der Elektroden für die Anregung und Messung indem sie den elektrischen Kontakt zwischen einer definierten Auswahl von Elektroden und einer Reihe von Messleitungen herstellt. Die Messleitungen sind an einem geeigneten Impedanzanalysator oder ein Messgerät angeschlossen, welches die Anregung und Messung der Signale übernimmt und die Daten über eine Schnittstelle zur Verfügung stellt. Über die Datenleitungen werden die Messdaten an eine Steuer- und Recheneinheit weitergeleitet. In dieser erfolgt die Auswertung und Aufbereitung der Daten sowie deren Speicherung. Die Ergebnisse werden über Datenleitungen an die Schnittstelle geleitet. Über diese kann ein Bediener bzw. ein weiteres Analyse- oder Messsystem auf die Ergebnisse zugreifen. Außerdem ist es möglich von der Schnittstelle aus über Steuerleitungen Statuswerte der Einzelkomponenten des Messsystems abzurufen und/oder diese Komponenten zu steuern bzw. zu regeln. Beispielsweise kann über die Steuerleitungen durch den Multifunktions-Switch / die Schaltmatrix Einfluss auf die zur Messung verwendeten Elektrodenkombination genommen, bestimmte Parameter der Monitoringzelle, wie der Nährlösungstransport, variiert, die Messparameter des Impedanzanalysators / des Messgerätes eingestellt und kontrolliert sowie die Auswertung und Aufzeichnung der Daten in der Steuer- und Recheneinheit gesteuert werden. Alle genannten Komponenten können sowohl als einzelne Bestandteile des Systems als auch integriert in einem Komplettsystem angeordnet sein. Außerdem sind auch Systeme denkbar, die auf einen oder mehrere Schritte des dargestellten Schemas verzichten, solange das Monitoring dadurch nicht beeinträchtigt ist.

Verfahrensabläufe (workflows) typischer Anwendungsfälle wie der Generierung neuer Testmethodiken und der Erstellung von Testbibliotheken, sind nachfolgend erläutert. Die einzelnen Arbeitsschritte, Entscheidungen, Daten und Ergebnisse sind jeweils den Bereichen "Monitoringzelle", "Mikrobiologie" oder "Monitoring-Management" zugeordnet. Ziel des Vorgehens ist es, aus dem Vergleich von Daten der biophysikalischen / elektrischen Messungen mittels Monitoringzelle mit den Daten testbegleitender und abschließender mikrobiologischer Tests geeignete Testparameter und Abbruchkriterien für einen stand-alone-Betrieb der Monitoringzelle für einen spezifischen Untersuchungsgegenstand zu gewinnen. Diese werden als Korrelationsmuster in Form einer Signalbibliothek bzw. als Kriterien für den Testabbruch in Form einer Trigger-Bibliothek gesammelt und sind Grundlage für ein autonomes Monitoring.

Ausgehend von Initialwerten wird eine Testmethodik für einen spezifischen Untersuchungsgegenstand erstellt. Diese enthält neben unveränderlichen Parametern sowie Arbeits- und Verfahrensvorschriften für die ordnungsgemäße Durchführung der Messungen und Untersuchungen auch Initialwerte für variierbare Testparameter der testbegleitenden mikrobiologischen Tests, der biophysikalischen Messungen sowie Abbruchkriterien für das Monitoring. Mit den Initialwerten werden biophysikalische Messungen und testbegleitende mikrobiologische Tests gestartet.

In einem dauernden Abgleich entscheidet ein Trigger anhand aktueller Abbruchkriterien über die Fortführung oder den Abbruch des Monitorings. Parallel dazu werden die gerade aktuellen Daten der biophysikalischen Messungen und testbegleitenden mikrobiologischen Tests für die nachfolgende Auswertung gesammelt. Liefert der Trigger die Entscheidung, das Monitoring aufgrund erfüllter Abbruchkriterien zu beenden, werden abschließende mikrobiologische Tests durchgeführt und die gesammelten biophysikalischen Daten sowie die Daten der testbegleitenden und abschließenden mikrobiologischen Tests der Auswertung und einem Vergleich (matching) zugeführt und alle relevanten Daten gespeichert.

Gefundene Korrelationsmuster zwischen gemessenen biophysikalischen und mikrobiologischen Parametern werden in eine Signalbibliothek, gefundene potenzielle Abbruchkriterien in eine Trigger-Bibliothek aufgenommen. Aus den neu gewonnenen Erkenntnissen werden Folgerung für den künftigen Testablauf der Messzelle im stand-alone-Betrieb abgeleitet. Gegebenenfalls wird ein Monitoring-Zyklus zur weiteren Verbesserung der Testmethodik oder der Generierung neuer Korrelationsmuster mit aktualisierten Abbruchkriterien und Testparametern erneut durchlaufen.

Der Prozess kann prinzipiell auch immer dann erneut durchlaufen werden, wenn eine Anpassung der Testmethodik notwendig wird oder neue Zellsubstrate und/oder Trägerobjekte zu untersuchen sind. Als Initialwerte einer neuen Testmethodik können dann die bereits gewonnenen Erkenntnisse in Gestalt einer bereits erstellten Signalbibliothek und Triggerbibliothek dienen.

Ein typischer workflow für den Anwendungsfall "Biophysikalisches Monitoring von Zellwachstumsprozessen", d.h. den "stand-alone"-Betrieb der Monitoringzelle ist nachfolgend erläutert, wobei die testbegleitenden mikrobiologischen Tests entfallen können. Als Ausgangspunkt dient die Trigger-Bibliothek. Aus dieser werden die für den Untersuchungsgegenstand relevanten Abbruchkriterien unter Zugrundelegung der zugehörigen Testmethodik entnommen. Anschließend wird die Monitoringzelle entsprechend konfiguriert und die biophysikalischen Messungen werden gestartet.

In einem dauernden Abgleich entscheidet ein Trigger anhand der festgelegten Abbruchkriterien über die Fortführung oder den Abbruch des Monitoring. Parallel dazu werden die gerade aktuellen biophysikalischen Daten für die nachfolgende Auswertung gesammelt. Liefert der Trigger die Entscheidung, das Monitoring aufgrund erfüllter Abbruchkriterien zu beenden, werden die biophysikalischen Daten in einem sogenannten Matching-Schritt mit Mustern aus der Signal-Bibliothek verglichen und ein Signal ("gematchtes" Signal) ausgegeben. Der Matching-Schritt dient dazu, aufgrund von Vergleichen und festgestellten Korrelationen von jeweils zu einem bestimmten Messzeitpunkten erfassten Messgrößen einen komplexen Zustand des analysierten Systems aus Trägerobjekt und Zellsubstrat zu diesem Messzeitpunkt darzustellen. Das erfindungsgemäße Verfahren kann daher als räumlich-zeitliche Abbildung des Prozesses des Wachstums der Zellen in Form elektrischer Signale verstanden werden, in denen die in der Monitoringzelle über eine Zeitdauer stattfindenden komplexen Prozesse integriert sind. Nach Ablegen der Signale als Daten in der Datenbank kann bei späteren Analysen auf diese Daten zugegriffen und zur Auswertung verwendet werden.

Dem Signal werden in einem weiteren Auswertungsschritt, gegebenenfalls aufgrund zusätzlicher Berechungen, die relevanten Zellwachstumsdaten zugeordnet und anschließend weiterverarbeitet. Alle relevanten Daten werden außerdem gespeichert. Der Ablauf kann nun mit veränderten Parametern (z. B. mit anderen Trägerobjekten) wiederholt werden. Auf diese Art kann eine alternative Vergleichsmöglichkeit von Zellwachstumsprozessen auf unterschiedlichen Trägerobjekten ohne den Einsatz mikrobiologischer Untersuchungen realisiert werden.

Das erfindungsgemäße Verfahren erlaubt es, wiederholte Messungen an einer bestimmten Kombination von Trägerobjekt / Zellsubstrat durchzuführen, ohne dabei zu analysierende Oberfläche der Trägerobjekte biologisch zu verändern oder zu beeinflussen. Es ist ermöglicht, gleiche Trägerobjekte mit unterschiedlichen Zellsubstraten zu kombinieren und aussagekräftige Vergleiche durchzuführen. Durch das erfindungsgemäße Verfahren ist eine sehr gute Reproduzierbarkeit der Analysen gewährleistet. Mit dem erfindungsgemäßen Verfahren ist ein biophysikalisch wirkendes, nicht-testabbrechendes Monitoringverfahren verfügbar, welches komplementär und komparativ zu bekannten mikrobiologischen Test- und Prüfverfahren benutzt werden kann. Es eignet sich um Bibliotheken zu erstellen und den Wachstumsverlauf zu dokumentieren, wobei auf zeit- und kostenaufwändige mikrobiologische Testverfahren verzichtet werden kann.

Die Erfindung wird nachfolgend anhand von Ausführungsbeispielen und Abbildungen näher erläutert. Es zeigen:
- Fig. 1: ein Oberteil einer ersten Ausführung einer erfindungsgemäßen Monitoringzelle in der Draufsicht von unten;
- Fig. 2: das Oberteil einer zweiten Ausführung der erfindungsgemäßen Monitoringzelle in Seitenansicht einer Schnittdarstellung entlang der Linie A-A;
- Fig. 3a: eine erste Ausführung der erfindungsgemäßen Monitoringzelle mit Ober- und Unterteil mit einer zentralen Elektrode im Oberteil;
- Fig. 3b: eine zweite Ausführung der erfindungsgemäßen Monitoringzelle mit einer zentralen Elektrode im Ober- und Unterteil;
- Fig. 4: eine erste Ausführung eines Elektrodenclusters als sich kreuzende Streifenelektroden;
- Fig. 5: eine zweite Ausführung eines Elektrodenclusters als Array von Punkt-Ring-Elektroden;
- Fig. 6: eine erste Ausführung eines Trägerobjektes mit durch eine Barriere getrennten Trägerteilflächen;

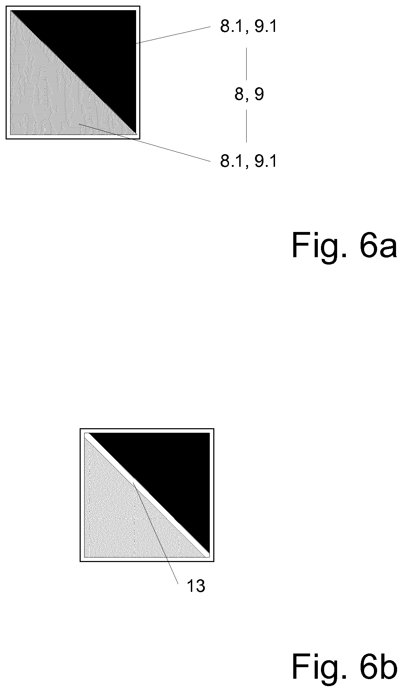

- Fig. 6: eine zweite Ausführung eines Trägerobjektes mit sich berührenden Trägerteilflächen ;

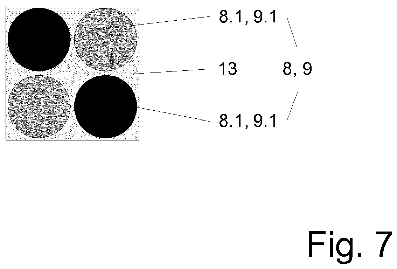

- Fig. 7: eine erste Ausführung eines Zellsubstrats mit voneinander durch bioinerte Bereiche getrennte Trägerteilflächen;

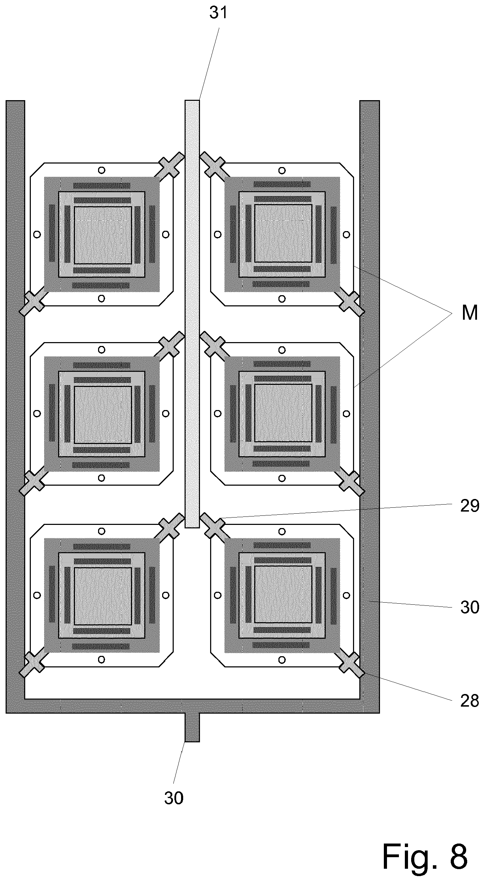

- Fig. 8: eine Anordnung von sechs Monitoringzellen in einer Draufsicht auf die Unterteile der Monitoringzellen;

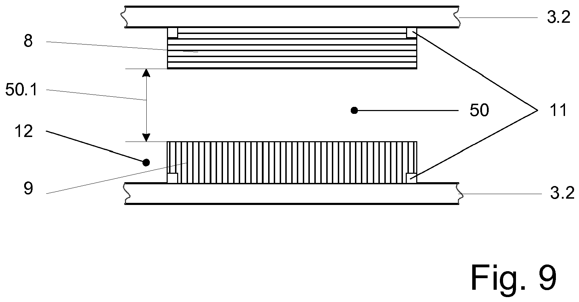

- Fig. 9: eine zweite Ausführung der erfindungsgemäßen Monitoringzelle.

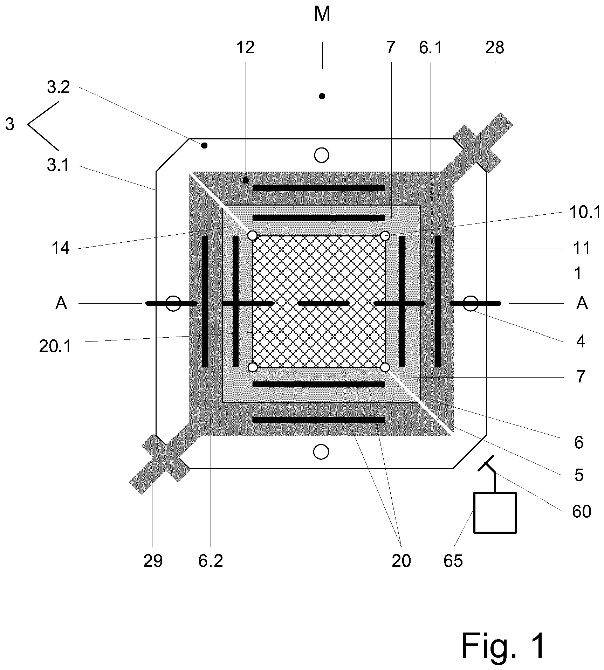

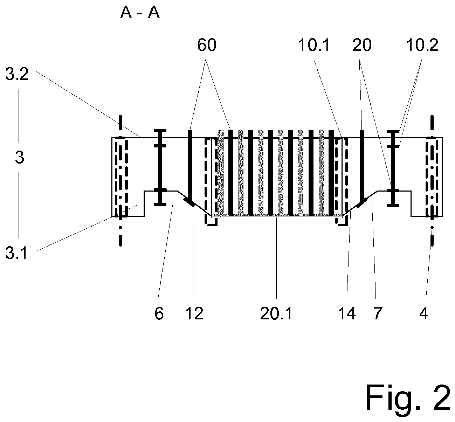

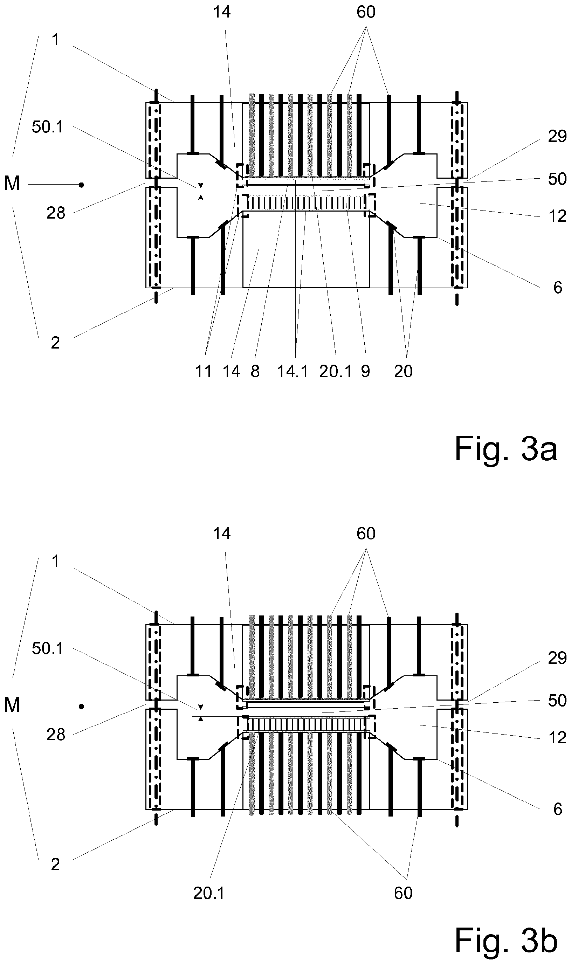

In Fig. 1 ist ein Oberteil 1 einer ersten Ausführung einer erfindungsgemäßen Monitoringzelle M gezeigt. Die wesentlichen Elemente sind ein Gehäuse 3 mit umlaufenden und auf den Betrachter zu ragenden Wänden 3.1 und einer Grundplatte 3.2, wobei durch Wände 3.1 und Grundplatte 3.2 ein nach oben offener Innenraum 12 mit quadratischer Grundfläche umfangen ist, ein von der Grundplatte 3.2 in den Innenraum 12 ragender Vorsprung 14, eine Aufnahmevorrichtung 11 zur Halterung und Fixierung eines Trägerobjekts 8 (siehe Fig. 3), ein zwischen dem Vorsprung 14 und den Wänden 3.1 gebildeter Strömungskanal 6, Elektroden 20 und eine Medienzuführung 28 und eine Medienabführung 29. Die Linie A - A bezeichnet den Verlauf einer Schnittlinie (siehe Fig. 2).

Ein nicht gezeigtes Unterteil 2 des ersten Ausführungsbeispiels der erfindungsgemäßen Monitoringzelle M ist identisch wie das Oberteil 1 aufgebaut. In den Wänden 3.1 sind im Oberteil 1 und Unterteil 2 Bohrungen 4 vorhanden, die zur flüssigkeitsdichten Verbindung von Oberteil 1 und Unterteil 2 mittels durch die Bohrungen 4 verlaufender Schraubverbindungen (nicht gezeigt) dienen. Der Vorsprung 14 ragt als Kegelstumpf mittig der Grundplatte 3.2 in den Innenraum 12 und weist seitliche, von der Grundplatte 3.2 ansteigende Flanken 7 und eine parallel der Grundplatte 3.2 verlaufende Stirnfläche 14.1 (siehe Fig. 3) auf. Auf einer der Diagonalen der Grundfläche des Innenraums 12 sind Strömungsbarrieren 5 von den Wänden 3.1 bis zur Stirnfläche 14.1 vorhanden, wodurch der Strömungskanal 6 in einen ersten Bereich 6.1 und einen zweiten Bereich 6.2 unterteilt ist. Auf der anderen Diagonale ist einmal die Medienzuführung 28 vorhanden, die mit dem ersten Bereich 6.1 strömungstechnisch in Verbindung steht und einmal die Medienabführung 29 vorhanden, die strömungstechnisch mit dem zweiten Bereich 6.2 in Verbindung steht.
Auf allen vier Seiten neben der Stirnfläche 14.1 sind als Streifenelektroden ausgebildete Elektroden 20 angeordnet. Jeweils eine Elektrode 20 befindet sich in dem Strömungskanal 6 und eine auf der Flanke 7, so dass insgesamt acht Elektroden 20 in bestimmter und bekannter Weise neben der Stirnfläche 14.1 angeordnet sind. Die gesamte Stirnfläche 14.1 ist von einer als Plattenelektrode gestalteten zentralen Elektrode 20.1 überdeckt. Diese zentrale Elektrode 20.1 ist über eine Höhenverstellung 10.1 in Richtung auf die Stirnfläche 14.1 verstellbar. Seitlich der zentralen Elektrode 20.1 ist die Aufnahmevorrichtung 11 angeordnet.

Alle Anschlussleitungen 60 der Elektroden 20 und der zentralen Elektrode 20.1 sind durch die Wände 3.1 oder durch die Grundplatte 3.2 verlaufend verlegt. Die Anschlussleitungen 60 dienen als Versorgungs- und Messdatenleitungen. Die Elektroden 20, 20.1 sind als sogenannte Aktor-Sensor-Systeme ausgebildet und einsetzbar. Sie sind insbesondere entsprechend eines solchen Systems verschalt- und ansteuerbar. Durch die Elektroden 20, 20.1 sind jeweils räumlich und zeitlich definiert elektrische Felder in dem Innenraum 12 erzeugbar (Aktor). Die Elektroden 20, 20.1 sind auch zur Erfassung elektrischer Messdaten geeignet (Sensor). Signaltechnisch verbunden sind die Elektroden 20, 20.1 mit einer Steuer- und Recheneinheit 65.

An der Höhenverstellung 10.1, in dem Innenraum 12 und/oder in oder an der Medienzuführung 28 und/oder der Medienabführung 29 können in weiteren Ausführungen der Monitoringzelle weitere Messmittel wie chemische Sensoren oder elektrische Sensoren vorhanden und signaltechnisch mit der Steuerung 65 verbunden sein.

In Fig. 2 ist ein Oberteil 1 einer zweiten Ausführung der erfindungsgemäßen Monitoringzelle M gezeigt, das die in Fig. 1 erläuterten Elementen aufweist und dessen zentrale Elektrode 20.1 als eine Anordnung (Array) von einzelnen Elektroden 20 als eine Clusterelektrode gestaltet ist. Die Anschlussleitungen 60 sind durch die Grundplatte 3.2 des Gehäuses 3 geführt. Stark schematisch dargestellt ist die Höhenverstellung 10.1. Die in dem Strömungskanal 6 angeordneten Elektroden 20 sind mit einer Höhenverstellung 10.2 verbunden und parallel der Wand 3.1 eine Strecke in den Innenraum 12 geschoben. Die auf den Flanken 7 angeordneten Elektroden 20 liegen an der Oberfläche des Vorsprungs 14 an.

Eine zusammengesetzte erfindungsgemäße Monitoringzelle M ist in Fig. 3a gezeigt. Das Oberteil 1 und das Unterteil 2 sind so aufeinander gestülpt, dass die jeweiligen Stirnflächen 14.1 einander gegenüber liegen. Auf der Stirnfläche 14.1 des Unterteils 2 ist ein Zellsubstrat 9 durch die Aufnahmevorrichtung 11 des Unterteils 2 gehalten und fixiert. Auf der Stirnfläche 14.1 des Oberteils 1 ist ein Trägerobjekt 8 durch die Aufnahmevorrichtung 11 des Oberteils 1 gehalten und fixiert. Die freien Oberflächen von Trägerobjekt 8 und Zellsubstrat 9 liegen sich gegenüber und sind durch einen Spalt 50 getrennt, der eine Spaltbreite 50.1 von 650 µm aufweist. Die Aufnahmevorrichtungen 11 sind höhenverstellbar gestaltet, so dass die Spaltbreite 50.1 einstellbar ist. Das Oberteil 1 und das Unterteil 2 weist Elektroden 20 Innenraum 12 auf. Zusätzlich ist eine zentrale Elektrode 20.1 in Form einer Clusterelektrode im Oberteil 1 vorhanden. Der Innenraum 12 ist von einem Medium ausgehend von der Medienzuführung 28 in Richtung Medienabführung 29 durchströmt. Dabei ist durch die Wände 3.1 und den Vorsprung 14 ein Strömungsweg gebildet, der entlang des Strömungskanals 6 und durch den Spalt 50 verläuft. In weiteren Ausführungen, in denen Strömungsbarrieren 5 angeordnet sind, ist der Strömungsweg teilweise oder gänzlich durch den Spalt 50 verlaufend.

Eine in Fig. 3b dargestellte Ausführung weist zusätzlich eine zentrale Elektrode im Unterteil 2 auf. Dadurch ist auch eine Messung zwischen den zentralen Elektroden 20.1 über die Spaltbreite 50.1 und durch Trägerobjekt 8 und Zellsubstrat 9 möglich.

Zwischen dem Oberteil 1 und dem Unterteil 2 können Dichtungen vorhanden sein (nicht gezeigt), außerdem können die sich berührenden Bereiche der Wand 3.1 anders, beispielsweise konisch oder abgestuft, gestaltet sein.

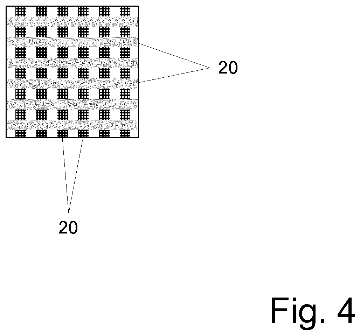

Ein erstes Ausführungsbeispiel einer als Clusterelektrode gemäß Fig. 4 ist aus elektrisch isolierten, sich orthogonal kreuzenden Elektroden 20 gebildet.

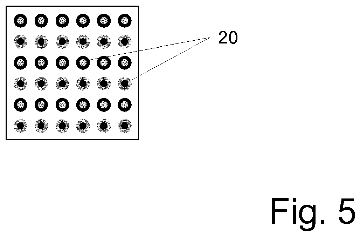

Die Fig. 5 zeigt ein zweites Ausführungsbeispiel einer Clusterelektrode, die aus einem Array von als Punkt-Ring-Elektroden ausgebildeten Elektroden 20 besteht. Die Elektroden sind in Zeilen angeordnet, wobei eine Polung der Elektroden 20 von Zeile zu Zeile alternierend wechselt.

In weiteren Ausführungen der erfindungsgemäßen Monitoringzelle kann eine Clusterelektrode auch als ganzflächige Elektrode oder als Potentialfeld messende Clusterelektrode aus Monopunktelektroden ausgebildet sein.

In Fig. 6a ist ein in Teilträgerobjekte 8.1 unterteiltes Trägerobjekt 8 gezeigt, dessen Teilträgerobjekte 8.1 direkt aneinander stoßen. Die Teilträgerobjekte 8.1 sind Materialien mit stark voneinander verschiedenen Oberflächenrauhigkeiten, wodurch eine Grenzflächensituation zwischen den Teilträgerobjekten 8.1 gegeben ist, die eine Spaltwirkung hat.

In Fig. 6b ist zwischen den Teilträgerobjekten 8.1 eine bioinerte, zytotoxisch beschichtete Barrierezone 13 vorhanden, die als Spalt 50 wirkt.

Die für die Fig. 6 und 7 gegebenen Beschreibungen können jeweils direkt auf Zellsubstrate 9 bzw. Trägerobjekte 8 übertragen werden, weshalb die Bezugszeichen doppelt vergeben sind.

Die Teilträgerobjekte 8.1 können auch eine in x- oder y -Richtung gradierte Oberfläche aufweisen.

In Fig. 7 sind Zellsubstrate 9 gezeigt, die in Teilzellsubstrate 9.1 gegliedert sind. Die Teilzellsubstrate 9.1 Die Teilzellsubstrate 9.1 sind rund und voneinander durch eine bioinerte, zytotoxisch beschichtete Barrierezone 13 getrennt, die als Spalt 50 wirkt.

In weiteren Ausführungen können die Unterteilungen auch in anderen Formen, Größen und Lagebeziehungen ausgebildet sein. Die Barrierezone 13 kann in weiteren Ausführungen auch neutral sein.

In einem nicht gezeigten Ausführungsbeispiel ist eine von einer Barriere 13 in einer Ebene ganz oder teilweise umgrenzte erste zentrale Elektrode 20.1 angeordnet über dem ein Zellsubstrat 9 vorhanden ist. Um die Barriere 13 ist ein ringförmiges Trägerobjekt 8 vorhanden. Gegenüber der zentralen Elektrode 20.1 ist eine zweite zentrale Elektrode 20.1 angeordnet, durch die zweite zentrale Elektrode 20.1 sind die erste zentrale Elektrode 20.1 mit Zellsubstrat 9, die Barriere 13 und das Trägerobjekt 8 in der Draufsicht überdeckt. Eine Messung erfolgt durch das Zellsubstrat 9 und / oder zwischen Zellsubstrat 9 und Trägerobjekt 8.

Mehrere der erfindungsgemäßen Monitoringzellen M können zusammengeschlossen sein, wie dies in Fig. 8 gezeigt ist. Analog zu sogenannten 6-well-Platten sind sechs Monitoringzellen M in zwei Spalten und drei Zeilen angeordnet. Jede Medienzuführung 28 jeder der Monitoringzellen M ist an eine Medienzuführungsleitung 30 und jede Medienabführung 29 jeder der Monitoringzellen M ist an eine Medienabführungsleitung 31 angeschlossen.

In einer in Fig. 9 gezeigten zweiten Ausführung der Monitoringzelle M sind keine Vorsprünge 14 vorhanden, sondern die Aufnahmevorrichtungen 11 sind jeweils an zwei parallel zueinander verlaufenden Grundplatten 3.2 so angeordnet, dass diese in den Innenraum 12 ragen. In einer der Aufnahmeeinrichtungen 11 ist das Trägerobjekt 8 und in der anderen Aufnahmevorrichtung 11 ist das Zellsubstrat 9 eingelegt und gehalten. Zwischen den dem Spalt 50 zugewandten Oberflächen des Trägerobjekts 8 und des Zellsubstrats 9 ist eine Spaltbreite von 1,5 mm eingestellt.

Erfindungsgemäß dient die o.g. in vitro Testanordnung einem nicht testabbrechenden, mehrdimensionalen Verfahren zum Monitoring von in vitro Zell- und Gewebewachstum in einer definierbaren Grenzflächensituation zwischen einem gewebeähnlichen Bereich und einem Bereich in Gestalt von degradierbaren bzw. nicht degradierbaren Implantatoberflächen und oder Gewebeersatzstoffen. Es kann also für das Monitoring von Wachstumsprozessen in einem in situ ähnlichen dreidimensionalen Zell- und Gewebebereich mit gegenüberliegenden Grenzschichten inklusive Spaltbereich verwendet werden.

### Bezugszeichen

- 1: Oberteil (der Monitoringzelle)
- 2: Unterteil (der Monitoringzelle)
- 3: Gehäuse
- 3.1: Wand (des Gehäuses 3)
- 3.2: Grundplatte
- A-A: Schnittebene
- 4: Verbindungsbohrungen
- 5: Strömungsbarriere
- 6: Strömungskanal
- 6.1: erster Bereich (des Strömungskanals 6)
- 6.2: zweiter Bereich (des Strömungskanals 6)
- 7: Flanke (des Vorsprungs 14.1)
- 8: Trägerobjekt
- 8.1: Teilträgerobjekte
- 9: Zellsubstrat
- 9.1: Teilzellsubstrat
- 10.1: Höhenverstellung (der zentralen Elektrode 20.1)
- 10.2: Höhenverstellung (der Elektrode 20)
- 11: Aufnahmevorrichtung
- 12: Innenraum
- 13: Barrierezone
- 14: Vorsprung
- 14.1: Stirnfläche (des Vorsprungs 14)
- 20: Elektrode
- 20.1: zentrale Elektrode
- 28: Medienzuführung
- 29: Medienabführung
- 30: Medienzuführungsleitung
- 31: Medienabführungsleitung
- 50: Spalt
- 50.1: Spaltbreite
- 60: Anschlussleitung
- 65: Steuer- und Recheneinheit

## Patentansprüche

1. Monitoringzelle zur Analyse eines Zell- und Gewebewachstums mit einem einen Innenraum umschließenden Gehäuse mit Wänden und mindestens einer Grundplatte, wobei das Gehäuse mit mindestens einer Medienzuführung an mindestens einer Seite des Innenraums und mindestens einer Medienabführung an mindestens einer anderen Seite des Innenraums und mit einer Anzahl von in dem Innenraum angeordneten Elektroden versehen ist, **dadurch gekennzeichnet, dass**
- mindestens eine Aufnahmevorrichtung (11) zur Aufnahme mindestens eines Trägerobjektes (8) und mindestens eine Aufnahmevorrichtung (11) zur Aufnahme mindestens eines Zellsubstrats (9) oder mindestens eine Aufnahmevorrichtung (11) zur Aufnahme mindestens eines Trägerobjektes (8) und mindestens eines Zellsubstrats (9),
- mindestens zum Startzeitpunkt der Analyse zwischen Trägerobjekt (8) und Zellsubstrat (9) ein Spalt (50) vorhanden ist, so dass eine in vitro abzubildende in vivo Situation zwischen einem zu besiedelnden Träger und einem Substrat nachgebildet ist und
- die Elektroden (20) sowohl zur Erzeugung räumlich und zeitlich definierter elektrischer und / oder elektromagnetischer Felder als auch als Mittel zur Messung elektrischer Größen ausgestaltet sind,
wobei eine Erzeugung räumlich definierter elektrischer und / oder elektromagnetischer Felder durch eine dreidimensionale Anordnung der Elektroden im Innenraum erzielt ist und eine zeitlich definierte Erzeugung elektrischer und / oder elektromagnetischer Felder durch eine Ansteuerbarkeit der Elektroden durch eine mit den Elektroden signaltechnisch verbundene Steuer- und Recheneinheit (65) erreicht ist und
die einzelnen Elektroden der Monitoringzelle mit einem Multifunktions-Switch oder mit einer Schaltmatrix als Schaltmittel verbunden sind und durch dieses Schaltmittel ein elektrischer Kontakt zwischen einer definierten Auswahl von Elektroden und einer Reihe von Messleitungen herstellt wird.

2. Monitoringzelle nach Anspruch 1, **dadurch gekennzeichnet, dass** in dem Innenraum zwei Vorsprünge mit Stirnflächen (14.1) vorhanden sind und die Aufnahmevorrichtungen (11) an den Stirnflächen (14.1) angeordnet sind.

3. Monitoringzelle nach Anspruch 1 oder 2, **dadurch gekennzeichnet, dass** eine Auswerte- und Speichereinheit (65) vorhanden ist, die mit den Elektroden (20) und weiteren Mitteln zur Messung physikalischer Größen verbunden ist.

4. Monitoringzelle nach einem der Ansprüche 1 bis 3, **dadurch gekennzeichnet, dass** in dem Innenraum (12) Strömungsbarrieren (5) angeordnet sind, durch deren Anordnung das Medium dem Spalt (50) zugeführt ist.

5. Monitoringzelle nach einem der vorhergehenden Ansprüche, **dadurch gekennzeichnet, dass** jeder Vorsprung (14) abfallende Flanken (7) aufweist.

6. Monitoringzelle nach einem der vorhergehenden Ansprüche, **dadurch gekennzeichnet, dass** die Monitoringzelle (M) mit einem gesteuerten Antrieb verbunden ist, mittels dem die Monitoringzelle (M) gesteuert bewegbar ist.

7. Monitoringzelle nach einem der vorhergehenden Ansprüche, **dadurch gekennzeichnet, dass** die Aufnahmevorrichtungen (11) verstellbar sind, so dass eine Spaltbreite (50.1) des Spalts (50) einstellbar ist.

8. Monitoringzelle nach Anspruch 7, **dadurch gekennzeichnet, dass** die Spaltbreite (50.1) kleiner als 3 mm ist.

9. Monitoringzelle nach einem der vorhergehenden Ansprüche, **dadurch gekennzeichnet, dass** die Elektroden (20) auf mindestens zwei Seiten neben den Stirnflächen (14.1) der Vorsprünge (14) angeordnet sind.

10. Monitoringzelle nach einem der vorhergehenden Ansprüche, **dadurch gekennzeichnet, dass** mindestens eine Elektrode (20) als zentrale Elektrode (20.1) auf mindestens einer Stirnfläche (14.1) angeordnet ist.

11. Monitoringzelle nach einem der vorhergehenden Ansprüche, **dadurch gekennzeichnet, dass** das Trägerobjekt (8) in Teilträgerobjekte (8.1) unterteilt ist.

12. Monitoringzelle nach einem der vorhergehenden Ansprüche, **dadurch gekennzeichnet, dass** das Zellsubstrat (9) in Teilzellsubstrate (9.1) unterteilt ist.

13. Verfahren zum nicht-testabbrechenden, räumlich-zeitlichen in vitro - Monitoring des Wachstums von Zellen oder Geweben unter Verwendung einer Monitoringzelle nach einem der vorhergehenden Ansprüche, mit den Schritten:
- Aufnehmen des Trägerobjekts (8) in der Aufnahmevorrichtung (11) der Monitoringzelle (M),
- Aufnehmen des Zellsubstrats (9) in einer weiteren Aufnahmevorrichtung (11) der Monitoringzelle (M), so dass zwischen dem Trägerobjekt (8) und dem Zellsubstrat (9) ein Spalt (50) mit einer Spaltbreite (50.1) vorliegt,
- Zuführen eines Mediums,
- Erzeugen räumlich und zeitlich definierter elektrischer und / oder elektromagnetischer Felder in der Monitoringzelle (M) und
- Erfassen und Speichern von Messgrößen der elektrischen und / oder elektromagnetischen Felder.

14. Verfahren nach Anspruch 13, **dadurch gekennzeichnet, dass** das Medium regelmäßig erneuert wird.

15. Verfahren nach Anspruch 13 oder 14, **dadurch gekennzeichnet, dass** verschiedene Medien gleichzeitig oder nacheinander zugeführt werden.

16. Verfahren nach einem der Ansprüche 13 bis 15, **dadurch gekennzeichnet, dass** initiale Messgrößen zu einem initialen Messzeitpunkt erfasst und gespeichert werden und die initialen Messgrößen als Referenzmessgrößen für zu späteren Messzeitpunkten erfassten Messgrößen verwendet werden.

17. Verfahren nach einem der Ansprüche 13 bis 16, **dadurch gekennzeichnet, dass** aus den Veränderungen der zu verschiedenen Messzeitpunkten erfassten und gespeicherten Messgrößen Informationen über das Wachstum der Zellen oder Gewebe abgeleitet werden.

18. Verfahren nach einem der Ansprüche 13 bis 17, **dadurch gekennzeichnet, dass** die Messgrößen ortsaufgelöst erfasst werden.

19. Verfahren nach Anspruch 18, **dadurch gekennzeichnet, dass** die ortsaufgelöst erfassten Messdaten graphisch dargestellt werden und die Informationen über das Wachstum der Zellen oder Gewebe aus der graphischen Darstellung abgeleitet werden.

20. Verfahren nach einem der Ansprüche 13 bis 19, **dadurch gekennzeichnet, dass** die Informationen mit einer Datenbank abgeglichen werden.

21. Verfahren nach einem der Ansprüche 13 bis 20, **dadurch gekennzeichnet, dass** das Wachstum der Zellen oder Gewebe durch eine definierte Einbringung von chemischen Substanzen in den Innenraum (12) beeinflusst wird.

22. Verfahren nach einem der Ansprüche 13 bis 21, **dadurch gekennzeichnet, dass** das Wachstum der Zellen oder Gewebe durch eine definierte Einkopplung physikalischer Wirkfaktoren in den Innenraum (12) beeinflusst wird.

23. Verfahren nach einem der Ansprüche 13 bis 22, **dadurch gekennzeichnet, dass** jede Spaltbreite kleiner als 3 mm eingestellt werden kann.

## Claims

1. A monitoring cell for the analysis of cell and tissue growth, with a housing enclosing an interior space with walls and at least one base plate, wherein the housing is provided with at least one media supply on at least one side of the interior space and at least one media discharge on at least one other side of the interior space and with a number of electrodes arranged in the interior space, **characterised in that**
- at least one receiving device (11) for receiving at least one carrier object (8) and at least one receiving device (11) for receiving at least one cell substrate (9) or at least one receiving device (11) for receiving at least one carrier object (8) and at least one cell substrate (9) are provided,
- a gap (50) is present between the carrier object (8) and the cell substrate (9) at least at the start time of the analysis, so that an in vivo situation to be imaged in vitro is simulated between a carrier to be colonized and a substrate, and
- the electrodes (20) are designed both for generating spatially and temporally defined electric and/or electromagnetic fields and also as means for measuring electrical quantities,
wherein the generation of spatially defined electric and/or electromagnetic fields is achieved by a three-dimensional arrangement of the electrodes in the interior space, and a temporally defined generation of electric and/or electromagnetic fields is achieved by the electrodes being controllable by a control and computing unit (65) connected to the electrodes in terms of signal technology, and
the individual electrodes of the monitoring cell are connected to a multifunction switch or to a switching matrix as switching means and this switching means establishes an electrical contact between a defined selection of electrodes and a series of measuring lines.

2. The monitoring cell according to claim 1, **characterised in that** two projections with end faces (14.1) are present in the interior space and the receiving devices (11) are arranged on the end faces (14.1).

3. The monitoring cell according to claim 1 or 2, **characterised in that** an evaluation and storage unit (65) is present which is connected to the electrodes (20) and further means for measuring physical quantities.

4. The monitoring cell according to any one of claims 1 to 3, **characterised in that** flow barriers (5) are arranged in the interior space (12), through the arrangement of which the medium is supplied to the gap (50).

5. The monitoring cell according to any one of the preceding claims, **characterised in that** each projection (14) has chamfered edges (7).

6. The monitoring cell according to any one of the preceding claims, **characterised in that** the monitoring cell (M) is connected to a controlled drive by means of which the monitoring cell (M) can be moved in a controlled manner.

7. The monitoring cell according to any one of the preceding claims, **characterised in that** the receiving devices (11) are adjustable so as to adjust a gap width (50.1) of the gap (50).

8. The monitoring cell according to claim 7, **characterised in that** the gap width (50.1) is less than 3 mm.

9. The monitoring cell according to any one of the preceding claims, **characterised in that** the electrodes (20) are arranged next to the end faces (14.1) of the projections (14) on at least two sides.

10. The monitoring cell according to any one of the preceding claims, **characterised in that** at least one electrode (20) is arranged as the central electrode (20.1) on at least one end face (14.1).

11. The monitoring cell according to any one of the preceding claims, **characterised in that** the carrier object (8) is divided into sub-carrier objects (8.1).

12. The monitoring cell according to any one of the preceding claims, **characterised in that** the cell substrate (9) is divided into sub-cell substrates (9.1).

13. A method for non-test terminating, spatio-temporal in vitro monitoring of the growth of cells or tissues using a monitoring cell according to any one of the preceding claims, comprising the steps of:
- receiving the carrier object (8) in the receiving device (11) of the monitoring cell (M),
- receiving the cell substrate (9) in a further receiving device (11) of the monitoring cell (M), so that there is a gap (50) with a gap width (50.1) between the carrier object (8) and the cell substrate (9),
- supplying a medium,
- generating spatially and temporally defined electric and / or electromagnetic fields in the monitoring cell (M), and
- capturing and storing measured variables of the electric and / or electromagnetic fields.

14. The method according to claim 13, **characterised in that** the medium is regularly renewed.

15. The method according to claim 13 or 14, **characterised in that** different media are supplied simultaneously or successively.

16. The method according to any one of claims 13 to 15, **characterised in that** initial measured variables are captured and stored at an initial measurement time and the initial measured variables are used as reference measured variables for measured variables captured at later measurement times.

17. The method according to any one of claims 13 to 16, **characterised in that** information about the growth of the cells or tissues is derived from the changes in the measured variables captured and stored at different measurement times.

18. The method according to any one of claims 13 to 17, **characterised in that** the measured variables are captured in a space-resolved manner.

19. The method according to claim 18, **characterised in that** the measurement data captured in a space-resolved manner are graphically represented and the information about the growth of the cells or tissues is derived from the graphical representation.

20. The method according to any one of claims 13 to 19, **characterised in that** the information is matched with a database.

21. The method according to any one of claims 13 to 20, **characterised in that** the growth of the cells or tissues is influenced by a defined introduction of chemical substances into the interior space (12).

22. The method according to any one of claims 13 to 21, **characterised in that** the growth of the cells or tissues is influenced by a defined coupling of physical action factors into the interior space (12).

23. The method according to any one of claims 13 to 22, **characterised in that** each gap width can be set to less than 3 mm.

## Revendications

1. Cellule de surveillance pour l'analyse de la croissance cellulaire et tissulaire avec un boîtier entourant un espace intérieur présentant des parois et au moins une plaque de base, le boîtier étant pourvu d'au moins une alimentation en fluide sur au moins un côté de l'espace intérieur et d'au moins une décharge de fluide sur au moins un autre côté de l'espace intérieur et avec plusieurs électrodes disposées dans l'espace intérieur, **caractérisée en ce que**
- l'on prévoit au moins un dispositif de réception (11) pour recevoir au moins un objet de support (8) et au moins un dispositif de réception (11) pour recevoir au moins un substrat cellulaire (9) ou au moins un dispositif récepteur (11) pour recevoir au moins un objet de support (8) et au moins un substrat cellulaire (9),
- un interstice (50) est présent entre l'objet de support (8) et le substrat cellulaire (9) au moins au moment du début de l'analyse, de sorte qu'une situation in vivo à visualiser in vitro est simulée entre un support à coloniser et un substrat, et
- les électrodes (20) sont réalisées à la fois pour générer des champs électriques et/ou électromagnétiques définis dans l'espace et dans le temps et également comme moyens de mesure de grandeurs électriques,
un engendrement de champs électriques et/ou électromagnétiques définis dans l'espace étant réalisé par une disposition tridimensionnelle des électrodes dans l'espace intérieur, et un engendrement de champs électriques et/ou électromagnétiques définis dans le temps étant réalisé à cause d'une contrôlabilité des électrodes par une unité de commande et de calcul (65) reliée aux électrodes du point de vue de la technique de signalisation, et
les différentes électrodes de la cellule de surveillance sont reliées à un commutateur multifonctions ou à une matrice de commutation comme moyen de commutation et ce moyen de commutation établit un contact électrique entre une sélection définie d'électrodes et une série de lignes de mesure.

2. Cellule de surveillance selon la revendication 1, **caractérisée en ce que** deux saillies avec des faces d'extrémité (14.1) sont présentes dans l'espace intérieur et les dispositifs de réception (11) sont disposés sur les faces d'extrémité (14.1).

3. Cellule de surveillance selon la revendication 1 ou 2, **caractérisée en ce qu'**il existe une unité d'évaluation et de mémorisation (65) qui est reliée aux électrodes (20) et d'autres moyens pour mesurer des grandeurs physiques.

4. Cellule de surveillance selon l'une quelconque des revendications 1 à 3, **caractérisée en ce que** des barrières d'écoulement (5) sont disposées dans l'espace intérieur (12), à travers lesquelles le fluide est amené vers l'interstice (50).

5. Cellule de surveillance selon l'une quelconque des revendications précédentes, **caractérisée en ce que** chaque saillie (14) présente des bords chanfreinés (7).

6. Cellule de surveillance selon l'une quelconque des revendications précédentes, **caractérisée en ce que** la cellule de surveillance (M) est reliée à un entraînement commandé au moyen duquel la cellule de surveillance (M) peut être déplacée de manière contrôlée.

7. Cellule de surveillance selon l'une quelconque des revendications précédentes, **caractérisée en ce que** les dispositifs de réception (11) sont réglables de telle sorte qu'une largeur d'interstice (50.1) de l'interstice (50) soit réglable.

8. Cellule de surveillance selon la revendication 7, **caractérisée en ce que** la largeur d'interstice (50.1) est inférieure à 3 mm.

9. Cellule de surveillance selon l'une quelconque des revendications précédentes, **caractérisée en ce que** les électrodes (20) sont disposées, sur au moins deux côtés, près des faces d'extrémité (14.1) des saillies (14).

10. Cellule de surveillance selon l'une quelconque des revendications précédentes, **caractérisée en ce qu'**au moins une électrode (20) est disposée comme électrode centrale (20.1) sur au moins une face d'extrémité (14.1).

11. Cellule de surveillance selon l'une quelconque des revendications précédentes, **caractérisée en ce que** l'objet de support (8) est divisé en sous-objets de support (8.1).

12. Cellule de surveillance selon l'une quelconque des revendications précédentes, **caractérisée en ce que** le substrat cellulaire (9) est divisé en sous-substrats cellulaires (9.1).

13. Procédé de surveillance in vitro spatio-temporelle, sans interruption d'essai, de la croissance de cellules ou de tissus en utilisant une cellule de surveillance selon l'une quelconque des revendications précédentes, comprenant les étapes consistant à :
- recevoir l'objet de support (8) dans le dispositif de réception (11) de la cellule de surveillance (M),
- recevoir le substrat cellulaire (9) dans un autre dispositif de réception (11) de la cellule de surveillance (M), de sorte qu'il existe un interstice (50) avec une largeur d'interstice (50.1) entre l'objet de support (8) et le substrat cellulaire (9),
- apporter un fluide,
- générer des champs électriques et/ou électromagnétiques définis dans l'espace et dans le temps dans la cellule de surveillance (M) et
- saisir et mémoriser des mesurandes des champs électriques et/ou électromagnétiques.

14. Procédé selon la revendication 13, **caractérisé en ce que** le fluide est régulièrement renouvelé.

15. Procédé selon la revendication 13 ou 14, **caractérisé en ce que** différents fluides sont fournis simultanément ou successivement.

16. Procédé selon l'une quelconque des revendications 13 à 15, **caractérisé en ce que** des mesurandes initiales sont saisies et mémorisées à un instant de mesure initiaux et les mesurandes initiaux sont utilisées comme mesurandes de référence pour des mesurandes saisis ultérieurement.

17. Procédé selon l'une quelconque des revendications 13 à 16, **caractérisé en ce que** des informations sur la croissance des cellules ou des tissus sont dérivées des modifications des grandeurs mesurées saisis et mémorisés à différents moments de mesure.

18. Procédé selon l'une quelconque des revendications 13 à 17, **caractérisé en ce que** les mesurandes sont saisis avec résolution spatiale.

19. Procédé selon la revendication 18, **caractérisé en ce que** les données de mesure saisies avec résolution spatiale sont représentées graphiquement et les informations sur la croissance des cellules ou tissus sont dérivées de la représentation graphique.

20. Procédé selon l'une quelconque des revendications 13 à 19, **caractérisé en ce que** les informations sont comparées à une base de données.

21. Procédé selon l'une quelconque des revendications 13 à 20, **caractérisé en ce que** la croissance des cellules ou tissus est influencée par une introduction définie de substances chimiques dans l'espace intérieur (12).

22. Procédé selon l'une quelconque des revendications 13 à 21, **caractérisé en ce que** la croissance des cellules ou tissus est influencée par un couplage défini de facteurs performants physiques dans l'espace intérieur (12).

23. Procédé selon l'une quelconque des revendications 13 à 22, **caractérisé en ce que** toute largeur d'interstice peut être réglée à moins de 3 mm.
